# EUROPEAN PATENT APPLICATION

(11) **EP 4 249 513 A2**
(43) Date of publication of application: **27.09.2023**
(21) Application number: 23185429.0
(22) Date of filing: 18.12.2019
(51) Int. Cl.: C07K 16/28

(54) **COMBINATIONS OF A HDM2-P53 INTERACTION INHIBITOR AND A BCL2 INHIBITOR AND THEIR USE FOR TREATING CANCER**

(30) Priority: 20.12.2018 US 201862782727 P; 20.12.2018 US 201862782730 P; 20.12.2018 US 201862782735 P
(62) Divisional of application: 19835803.8
(71) Applicant: Novartis AG, 4056 Basel (CH)
(72) Inventor: GUERREIRO, Nelson, 4002 Basel (CH); HALILOVIC, Ensar, Cambridge, 02139 (US); JULLION, Astrid, 4002 Basel (CH); MEILLE, Christophe, 4002 Basel (CH); WANG, Youzhen, Cambridge, 02139 (US); FABRE, Claire, 4002 Basel (CH)
(74) Representative: Ridout, Joseph

(57) **Abstract**

The present invention relates to the combination of the HDM2-p53 interaction inhibitor drug (S)-5-(5-Chloro-1-methyl-2-oxo-1,2-dihydro-pyridin-3-yl)-6-(4-chloro-phenyl)-2-(2,4-dimethoxy-pyrimidin-5-yl)-1-isopropyl-5,6-dihydro-1H-pyrrolo[3,4-d]imidazol-4-one [HDM201] and the BCL2 inhibitor 4-(4-{[2-(4-chlorophenyl)-4,4-dimethylcyclohex-1-en-1-yl]methyl}piperazin-1-yl)-N-[(3-nitro-4-{[(oxan-4yl)methyl]amino}phenyl)sulfonyl]-2-[(1H-pyrrolo[2,3-b]pyridin-5-yl)oxy]benzamide [venetoclax]. The present invention further relates to the use of said combination in the treament of cancer, in particular hematological tumors. The present invention further relates to dose and dosing regimen related to this combination cancer treatment.

## Description

### FIELD OF THE INVENTION

The present invention relates to the combination of the HDM2-p53 interaction inhibitor drug (S)-5-(5-Chloro-1-methyl-2-oxo-1,2-dihydro-pyridin-3-yl)-6-(4-chloro-phenyl)-2-(2,4-dimethoxy-pyrimidin-5-yl)-1-isopropyl-5,6-dihydro-1H-pyrrolo[3,4-d]imidazol-4-one [HDM201] and the BCL2 inhibitor 4-(4-{[2-(4-chlorophenyl)-4,4-dimethylcyclohex-1-en-1-yl]methyl}piperazin-1-yl)-N-[(3-nitro-4-{[(oxan-4yl)methyl]amino}phenyl)sulfonyl]-2-[(1H-pyrrolo[2,3-b]pyridin-5-yl)oxy]benzamide [venetoclax]. The present invention further relates to the use of said combination in the treatment of cancer, in particular hematological tumors. The present invention further relates to dose and dosing regimen related to this combination cancer treatment.

### BACKGROUND OF THE INVENTION

### HDM201

p53 is induced and activated by a number of potentially tumorigenic processes - including aberrant growth signals, DNA damage, ultraviolet light, and protein kinase inhibitors (Millard M, et al. Curr Pharm Design 2011;17:536-559) - and regulates genes controlling cell growth arrest, DNA repair, apoptosis, and angiogenesis (Bullock AN & Fersht AR. Nat Rev Cancer 2001;1:68-76; Vogelstein B, et al. Nature Education 2010;3(9):6).

Human Double Minute-2 (HDM2) is one of the most important regulators of p53. It binds directly to p53, inhibiting its transactivation, and subsequently directing it towards cytoplasmic degradation (Zhang Y, et al. Nucleic Acids Res 2010;38:6544-6554).

p53 is one of the most frequently inactivated proteins in human cancer, either through direct mutation of the *TP53* gene (found in approximately 50% of all human cancers) (Vogelstein, B et al. Nature 2000;408:307-310) or via suppressive mechanisms such as overexpression of HDM2 (Zhao Y, et al. BioDiscovery 2013;8:4)*.*

Potent and selective inhibitors of the HDM2-p53 interaction (also referred to as HDM2 inhibitors or MDM2 inhibitors), e.g. NVP-HDM201 (herein referred to as HDM201), have been shown to restore p53 function in preclinical cell and in vivo models (Holzer P, et al. Poster presented at AACR 2016, Abstract #4855, Holzer P, Chimia 2017, 71(10), 716-721). The HDM2 inhibitor HDM201, i.e. (S)-5-(5-Chloro-1-methyl-2-oxo-1,2-dihydro-pyridin-3-yl)-6-(4-chloro-phenyl)-2-(2,4-dimethoxy-pyrimidin-5-yl)-1-isopropyl-5,6-dihydro-1H-pyrrolo[3,4-d]imidazol-4-one, and methods how to prepare it were disclosed for example in WO2013/111105.

Different dosing regimens were described for HDM2 inhibitors and tested in clinical studies. E.g. US2013/0245089 discloses a method of treating a patient suffering from cancer by administering to the patient 4-{[(2R,3S,4R,5S)-4-(4-Chloro-2-fluoro-phenyl)-3-(3-chloro-2-fluoro-phenyl)-4-cyano-5-(2, 2-dimethyl-propyl)-pyrrolidine-2-carbonyl]-amino}-3-methoxybenzoic acid in an amount of from about 800 to about 3000 mg/day for an administration period of up to about 7 days, on days 1-7, of a 28 days treatment cycle, followed by a rest period of from about 21 to about 23 days.

A paper in Clinical Cancer Research by B. Higgins et al, in May 2014 (Higgins B. et al, Preclinical Optimisation of MDM2 Antagonist Scheduling for Cancer Treatment by Using a Model-Based Approach. Clin Cancer Research 2014; 20:3742-3752, disclosed a 28-day cycle schedule, where RG7388 is administered once weekly three times followed by 13 days of rest (28 days cycle schedule), or where the drug is administered for 5 consecutive days of a 28 days schedule.

Further dosing regimens for HDM2 inhibitors, e.g. intermittent high dose regimens and extended low dose regiments are disclosed in WO 2015/198266, WO 2018/092020, and WO 2018/178925.

However, long term platelet depletion and/or disease resistance limiting drug effect on bone marrow blasts in later treatment cycles is a common challenge in the therapies involving HMD2 inhibitors. Therefore, there remains a need for optimizing dose and regimens of these anti-cancer drugs to minimize the adverse effects.

### Venetoclax

Venetoclax is a potent, selective inhibitor of B-cell lymphoma (BCL)-2, an antiapoptotic protein. Venetoclax binds directly to the BH3-binding groove of BCL-2, displacing BH3 motif-containing pro-apoptotic proteins like BIM, to initiate mitochondrial outer membrane permeabilization (MOMP), caspase activation, and programmed cell death. Bcl-2 is an anti-apoptotic protein of the Bcl-2 family of proteins, which includes Mcl-1 and Bcl-xL, among others. Like Mcl-1 and Bcl-xL, Bcl-2 can bind pro-apoptotic effector molecules such as Bax/Bak and prevent their oligomerization and formation of the transmembrane pores on the mitochondrial membrane. By doing so, Bcl-2 prevents release of cytochrome C, thereby blocking activation of caspases and subsequently inhibiting apoptosis. Evasion of apoptosis is one of the hallmarks of cancers, thus the anti-apoptotic proteins such as Bcl-2, facilitators of this evasion, represent a compelling therapeutic target (Certo et al. 2006). Venetoclax is a highly selective orally bioavailable small-molecule inhibitor of Bcl-2 that has shown activity in Bcl-2 dependent leukemia and lymphoma cell lines (Souers et al. 2013). Venetoclax induces apoptotic cell death in AML/MDS cell lines, primary AML/MDS subject samples both in vitro, and in mouse xenograft models (Pan et al. 2014).

Venetoclax monotherapy has been approved by the FDA for the treatment of CLL/SLL (chronic lymphocytic leukemia/small lymphocytic lymphoma) with or without deletion 17p who have received at least one prior therapy [Venetoclax US PI]. In Europe, venetoclax has been approved by EMA for the treatment of CLL in the presence of 17p deletion or TP53 mutation in adult subjects who are unsuitable for or have failed a B-cell receptor pathway inhibitor; for the treatment of CLL in the absence of 17p deletion or TP53 mutation in adult subjects who have failed both chemo-immunotherapy and a B-cell receptor pathway inhibitor [Venetoclax EU SmPC].

Venetoclax is being studied in AMUMDS, in phase I/II and in phase III clinical trials in both AML subjects either unfit for standard induction chemotherapy or relapsed/refractory as well as high-risk MDS, both in monotherapy as well as in combination with standard of care and/or target therapies ([NCT01994837], [NCT02670044], [NCT03214562], [NCT02287233], [NCT03069352], [NCT02993523], [NCT02966782], [NCT02942290], and [NCT03404193]). As monotherapy, venetoclax has modest activity with CR/CRi rates of 19% (6/32 subjects) in R/R or unfit for chemotherapy AML subjects (Konopleva et al. 2016). In combination with hypomethylating agents or low dose aracytine (LDAC), CR/CRi rates of 59-62% are reported in AML subjects unfit for chemotherapy.

### Combination

Cancer monotherapies are often impacted by lack of sustained efficacy and/or safety issues. Combination cancer therapies based on combination partners which show a synergistic effect provide the advantage of substantially increased long term efficacy and improved safety profile. For this reason, it remains a desire to research for anti-cancer drugs combinations.

### SUMMARY OF THE INVENTION

A novel combination for cancer treatment has been found: the HDM2-p53 interaction inhibitor drug HDM201 and the BCL2 inhibitor venetoclax.

It has further been found that one type of dosing regimen is particularly useful for the treatment of hematological tumors with the HDM2 inhibitor HDM201 in combination with venetoclax.

Specifically, the present invention provides the following aspects, advantageous features and specific embodiments, respectively alone or in combination, as listed in the following embodiments:
1. The combination of the HDM2-p53 interaction inhibitor drug (S)-5-(5-Chloro-1-methyl-2-oxo-1,2-dihydro-pyridin-3-yl)-6-(4-chloro-phenyl)-2-(2,4-dimethoxy-pyrimidin-5-yl)-1-isopropyl-5,6-dihydro-1H-pyrrolo[3,4-d]imidazol-4-one [HDM201] or a pharmaceutically acceptable non-covalent derivative (including salt, solvate, hydrate, complex, co-crystal) thereof, and the BCL2 inhibitor 4-(4-{[2-(4-chlorophenyl)-4,4-dimethylcyclohex-1- en-1-yl]methyl}piperazin-1-yl)-N-[(3-nitro-4-{[(oxan-4yl)methyl]amino}phenyl)sulfonyl]-2-[(1H-pyrrolo[2,3-b]pyridin-5-yl)oxy]benzamide [venetoclax], or a pharmaceutically acceptable non-covalent derivative (including salt, solvate, hydrate, complex, co-crystal) thereof.
2. The combination according to embodiment 1 for use in the treatment of cancer.
3. The combination for use in the treatment of cancer according to embodiment 2, wherein the cancer is a hematological tumor.
4. The combination for use in the treatment of cancer according to embodiment 3, wherein the hematological tumor is acute myeloid leukemia (AML), preferably relapsed/refractory AML or first line (1L) AML (includes both *de novo* and secondary AML).
5. The combination for use in the treatment of cancer according to embodiment 3, wherein the hematological tumor is myelodysplastic syndrome (MDS), preferably high-risk MDS (including high and very high-risk MDS according to rIPSS (revised international prognostic scoring system)).
6. The combination for use in the treatment of cancer according to any one of embodiments 2 to 5, wherein the cancer is a TP53 wild-type tumor.
7. The combination for use in the treatment of cancer according to any one of embodiments 3 to 6,
   wherein HDM201 is administered on each of the first 3 to 7 days, preferably on each of the first 4 to 6 days, more preferably on each of the first 5 days, of a 28 days treatment cycle;
   wherein the HDM201 treatment is composed of at least three 28 days treatment cycles,
   wherein the HDM201 daily drug dose for the first and second treatment cycle (i.e. induction cycles) is from 50 mg to 100 mg, preferably from 50 mg to 80 mg, more preferably from 60 mg to 80 mg, even more preferably 60 mg, and the daily HDM201 dose for the third and any following treatment cycle (i.e. consolidation cycles) is from 10 mg to 45 mg, preferably from 20 mg to 40 mg, more preferably from 30 mg to 40 mg, even more preferably 40 mg.
8. The combination for use in the treatment of cancer according to any one of embodiments 3 to 6,
   wherein HDM201 is administered on each of the first 5 days of a 28 days treatment cycle,
   wherein the HDM201 treatment is composed of at least three 28 days treatment cycles, and
   wherein the daily HDM201 dose of the induction cycles (cycles 1 and 2) is from from 60 mg to 80 mg, and wherein the daily HDM201 dose of the consolidation cycles (cycles 3 and following) is 40 mg.
9. The combination for use in the treatment of cancer according to any one of embodiments 3 to 8,
   wherein venetoclax is administered with a daily dose of from 20 mg to 1000 mg, preferably from 50 mg to 600 mg, more preferably from 300 mg to 600 mg, even more preferably from 400 mg to 600 mg, even more preferably 400 mg or 600 mg.
10. The combination for use in the treatment of cancer according to any one of embodiments 3 to 6,
   wherein HDM201 is administered on each of the first 5 days of a 28 days treatment cycle, wherein the HDM201 treatment is composed of at least three 28 days treatment cycles, wherein the daily HDM201 dose of the induction cycles (cycles 1 and 2) is from from 60 mg to 80 mg, and wherein the daily HDM201 dose of the consolidation cycles (cycles 3 and following) is 40 mg, and
   wherein venetoclax is administered with a daily dose of 400 mg or 600 mg.
11. The combination or the combination for use in the treatment of cancer according to any one of the preceding embodiments, wherein HDM201, is present as non-covalent derivative, preferably said non-covalent derivative is selected from the group consisting of salt, solvate, hydrate, complex and co-crystal, more preferably the non-covalent derivative is a co-crystal, even more preferably present as succinic acid co-crystal, even more preferably as 1:1 (molar ratio) succinic acid : HDM201 co-crystal.
12. The combination or the combination for use in the treatment of cancer according to any one of the preceding embodiments, wherein the combination further comprises one or more other anti-cancer agents, preferably said anti-cancer agent(s) is(are) selected from: immuno-oncological drugs (e.g. PD-1 [e.g. PDR001(Novartis, INN Spartalizumab)], PD-L1, LAG-3, TIM-3 [e.g. MBG453(Novartis)], GTIR, TGF-beta, IL15 inhibitors), FLT3 inhibitors (e.g. gilterinib, quizartinib, midostaurin), BCL2 inhibitors (e.g. navitoclax, venetoclax), other HDM2 inhibitors (e.g. idasanutlin, AMG232, DS-3032B, ALRN6924/ATSP7041), hypomethylating agents (HMA) (e.g. Vidaza [azacytidine, 5-azacytidine], Dacogen [decitabine], guadecitabine), anthracyclines (e.g. idarubicin, daunorubicin, doxorubicin, epirubicin, rubidomycin); anti-CD33 antibodies (e.g. Mylotarg [gemtuzumab], vadastuximab) and other agents (e.g. AraC [cytarabine, aracytine]).
13. The combination or the combination for use in the treatment of cancer according to any one of the preceding embodiments, wherein the combination further comprises one or more other anti-cancer agents, preferably said anti-cancer agent(s) is(are) selected from: cytarabine (Ara-C), anthracycline, daunorubicin, idarubicin, rubidomycin, idamycin, midostaurin and azacytidine.

The combination therapy of the present invention provides the advantage of a substantially increased long term efficacy and an improved safety profile.

The dosing regimens of the present invention as described above provide a highly favorable therapeutic index, low incidence of grade 3/4 thrombocytopenia while achieving therapeutically relevant exposures, p53 pathway activation (GDF-15 upregulation), and clinical activity.

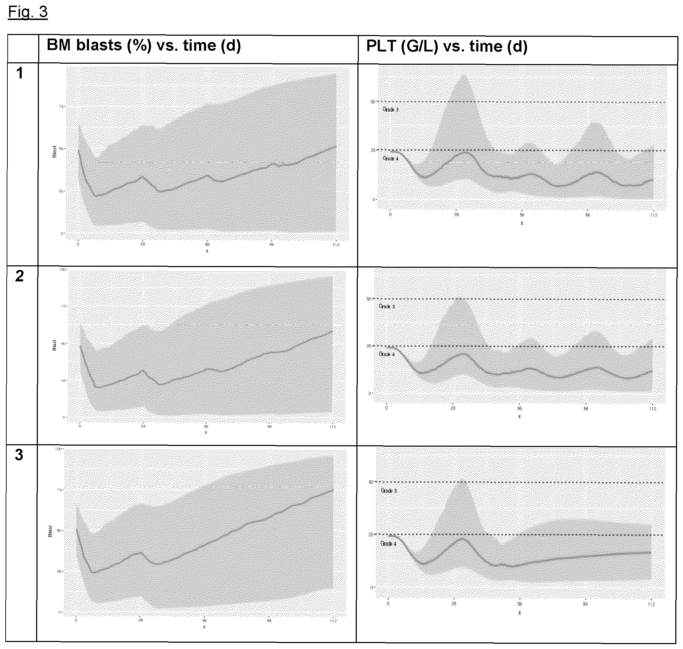

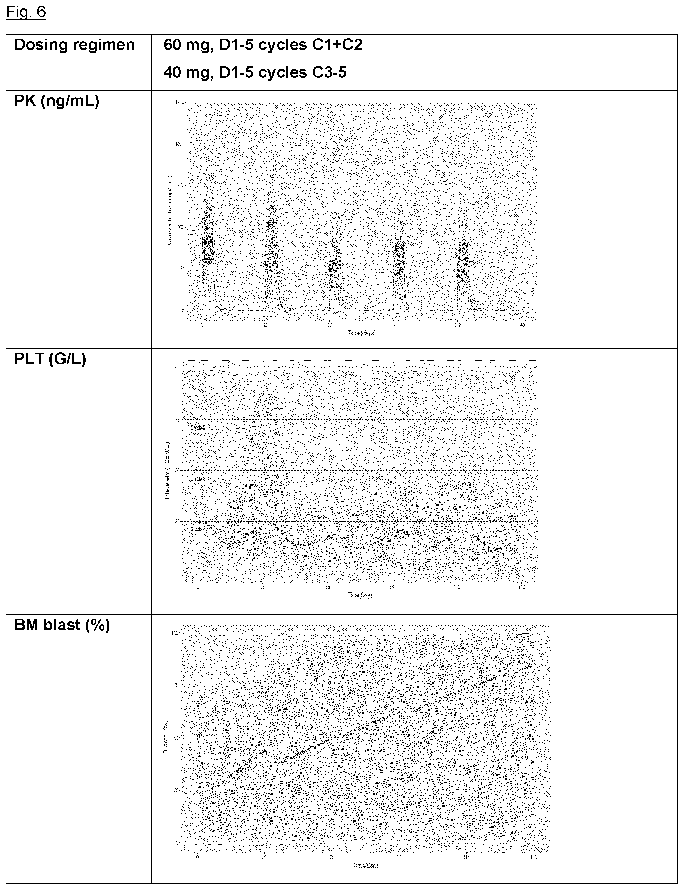

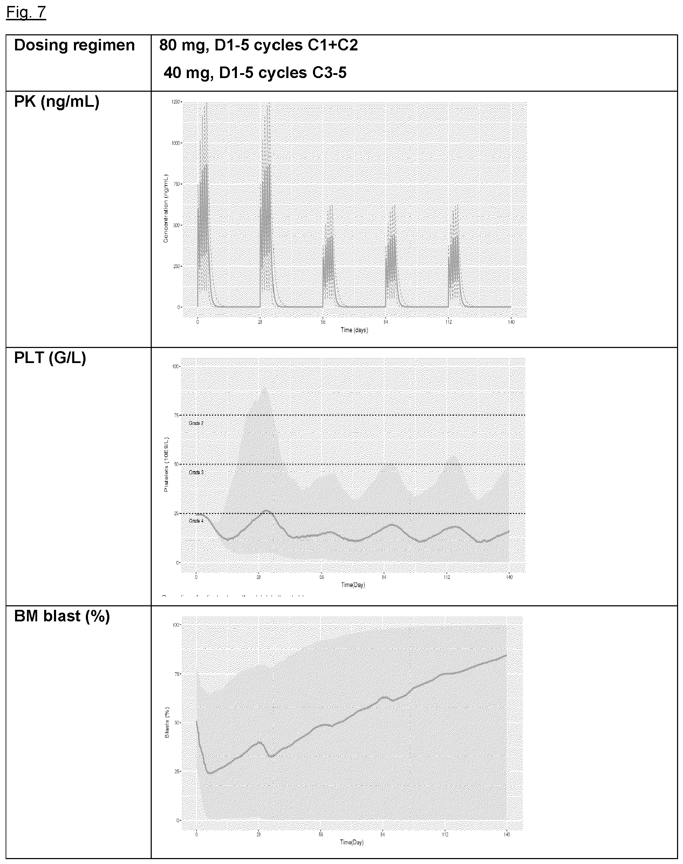

In particular, the dosing regimens of the present invention as described above provide a good bone marrow (BM) blasts response within the first two treatment cycles while managing effectively safety in subsequent treatment cycles (cycles 3 and following), see Figure 3, variant 2 and Figures 6-7.

### BRIEF DESCRIPTION OF THE DRAWINGS

In the following, the present invention is described in detail with reference to accompanying figures in which:

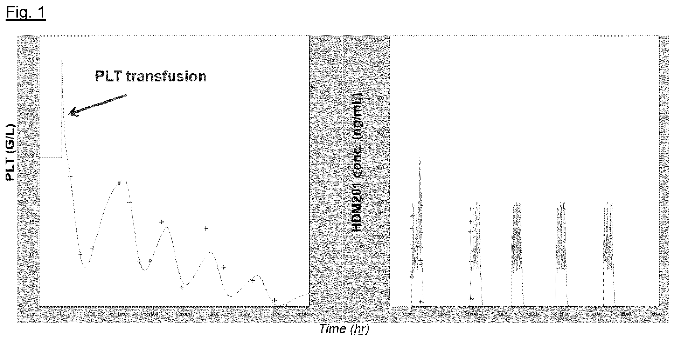

**Figure 1** shows an example of an individual platelet (PLT) profile (Regimen 2C, i.e. d1-7q28d, 45 mg), from clinical study CHDM201X2101.

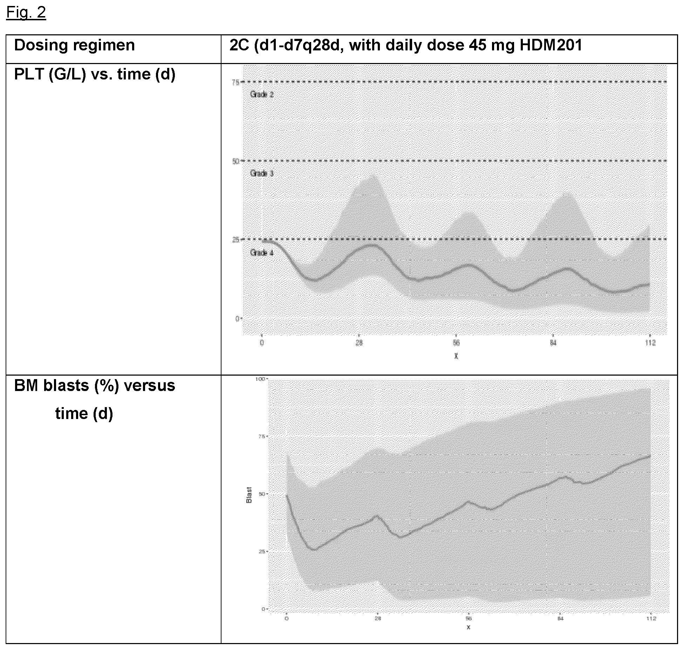

**Figure 2** shows the impact of dosing regimen 2C (d1-d7q28d, with daily dose 45 mg HDM201) on PLT profile is limited with no recovery. Long-term platelet depletion, PLT (G/L) versus time(d), Median and interquartile range. Figure 2 further shows the impact of dosing regimen on blast kinetics: regimen 2C with 45 mg daily dose HDM201 achieves good BM blasts depletion. Early and low nadir. BM blasts (%) versus time (d).
**Figure 3** shows the simulated profile for regiment 2C variants 1, 2, and 3. Variant 1: 60mg (4 cycles); Variant 2: 60mg (2 cycles) → 30mg (2 cycles); Variant 3: 60mg (2 cycles) → 0. Variants 2-3 provide dose(s) to maximize BM blasts response within first 2 cycles, while managing safety in subsequent cycles (cycles 3 and 4).

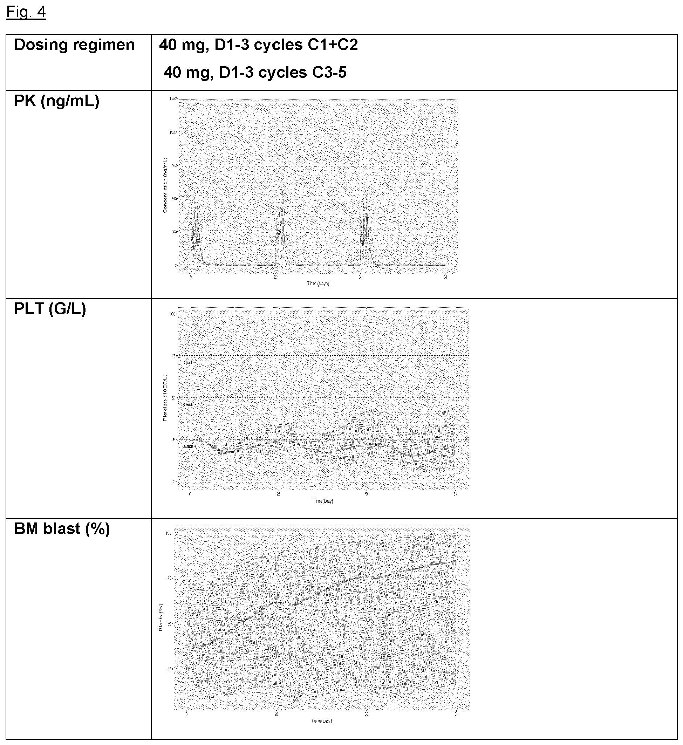

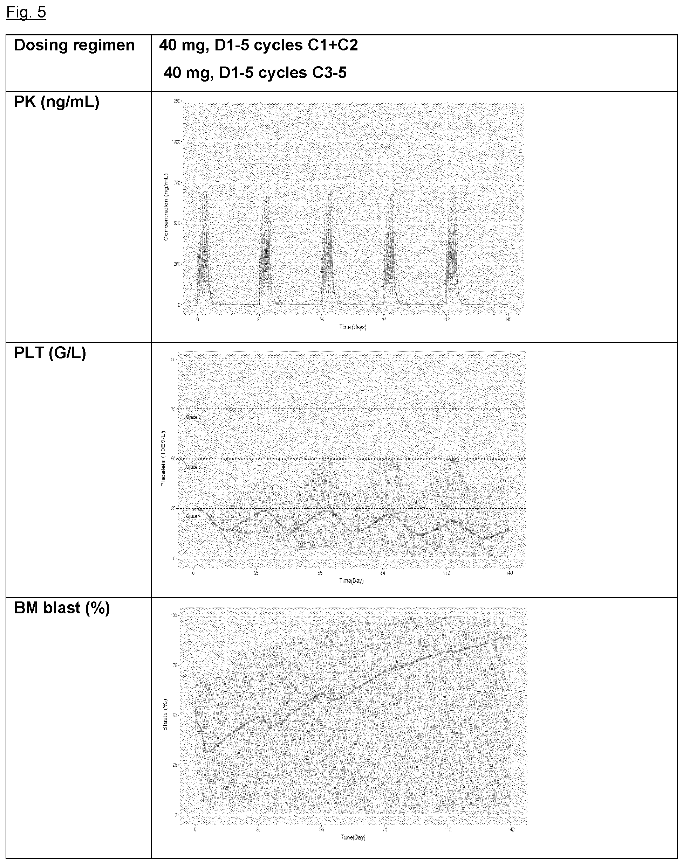

**Figures 4-7** shows the simulation of platelet (PLT) and bone marrow (BM) blast metrics from HDM201X2101 dose(s) to maximize BM blasts response within first 2 cycles, while managing safety in subsequent cycles (cycles 3-5)
**Figure 8****:** Summary of anti-leukemic effect of the HDM201 and Venetoclax combination:
   HDM201 enhances antitumor activity of a selective Bcl-2 inhibitor venetoclax in AML patient derived orthotopic model. NVP-HDM201 was administered po, at 20 mg/kg, 3 times on dosing day once weekly, either as a single agent or in combination with Venetoclax (ABT-199) po at 100mg/kg, once daily 5 times a week, for 14 days. Initial group size: 5 animals.
   (A) The mean leukemic burden is represented as CD45+ cells in peripheral blood for each treatment group, plotted against time for the 14 day treatment period.
   (B) Spleen weight and IHC staining of leukemic density in spleen. IHC analysis using the anti-human IDH1 mouse monoclonal primary Ab against IDH1R132H mutant protein. In the graphics (A) and (B), the order of the lines and dots follows the order in the legend (from top to bottom, from left to right).

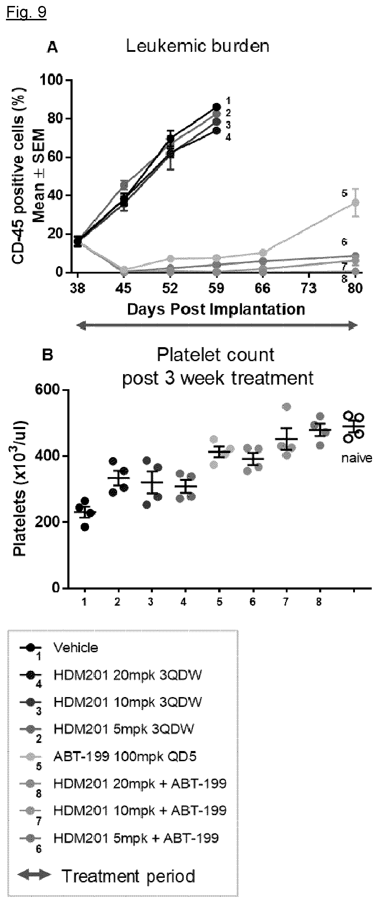

**Figure 9****:** Dose titration of HDM201 in combination with venetoclax:
   Effects of the combination on platelets could be mitigated with administration of lower doses of HDM201, while still maintaining the anti-tumor activity. NVP-HDM201 was administered po, at 5 mg/kg, 10 mg/kg, and 20 mg/kg, 3 times on dosing day once weekly, either as a single agent or in combination with Venetoclax (ABT-199) po at 100mg/kg, once daily 5 times a week, for 3-6 weeks. Initial group size: 4 animals.
   (A) The mean leukemic burden is represented as CD45+ cells in peripheral blood for each treatment group, plotted against time.
   (B) Platelet count was measured in peripheral blood after 3 weeks of treatment and depicted in panel B.

### DETAILED DESCRIPTION OF THE INVENTION

Herein after, the present invention is described in further detail and is exemplified.

### Definitions

Additional terms are defined below and throughout the application.

As used herein, the articles "a" and "an" refer to one or to more than one (e.g., to at least one) of the grammatical object of the article.

The term "or" is used herein to mean, and is used interchangeably with, the term "and/or," unless context clearly indicates otherwise.

"About" and "approximately" shall generally mean an acceptable degree of error for the quantity measured given the nature or precision of the measurements. Exemplary degrees of error are within 20 percent (%), typically, within 10%, and more typically, within 5% of a given value or range of values.
By "a combination" or "in combination with," it is not intended to imply that the therapy or the therapeutic agents must be administered at the same time and/or formulated for delivery together, although these methods of delivery are within the scope described herein. The therapeutic agents in the combination can be administered concurrently with, prior to, or subsequent to, one or more other additional therapies or therapeutic agents. The therapeutic agents or therapeutic protocol can be administered in any order. In general, each agent will be administered at a dose and/or on a time schedule determined for that agent. In will further be appreciated that the additional therapeutic agent utilized in this combination may be administered together in a single composition or administered separately in different compositions. In general, it is expected that additional therapeutic agents utilized in combination be utilized at levels that do not exceed the levels at which they are utilized individually. In some embodiments, the levels utilized in combination will be lower than those utilized individually.

HDM201 is defined by the following chemical names and structure.

HDM201:
(6S)-5-(5-chloro-1-methyl-2-oxo-1,2-dihydropyridin-3-yl)-6-(4-chlorophenyl)-2-(2,4-dimethoxypyrimidin-5-yl)-1-(propan-2-yl)-5,6-dihydropyrrolo[3,4-*d*]imidazol-4(1*H*)-one *antineoplastic*
(6*S*)-5-(5-chloro-1-methyl-2-oxo-1,2-dihydropyridin-3-yl)-6-(4-chlorophenyl)-2-(2,4-dimethoxypyrimidin-5-yl)-1-(propan-2-yl)-5,6-dihydropyrrolo[3,4-*d*]imidazol-4(1*H*)-one *antinéoplasique*
(6*S*)-5-(5-cloro-1-metil-2-oxo-1,2-dihidropiridin-3-il)-6-(4-clorofenil)-2-(2,4-dimetoxipirimidin-5-il)-1-(propan-2-il)-5,6-dihidropirrolo[3,4-*d*]imidazol-4(1*H*)-ona *antineoplásico*

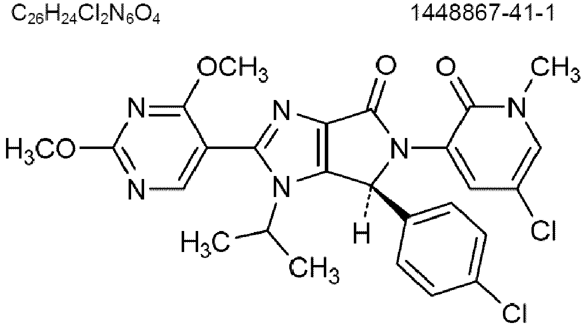

Venetoclax is defined by the follwing chemical names and structure.
venetoclaxum

| | |
|---|---|
| venetoclax | 4-(4-{[2-(4-chlorophenyl)-4,4-dimethylcyclohex-1-en-1-yl]methyl}piperazin-1-yl)-*N*-[(3-nitro-4-{[(oxan-4-yl)methyl]amino}phenyl)sulfonyl]-2-[(1*H*-pyrrolo[2,3-b]pyridin-5-yl)oxy]benzamide |
| vénétoclax | 4-(4-{[2-(4-chlorophenyl)-4,4-dim6thylcyclohex-1-6n-1-yl]méthyl}pipérazin-1-yl)-*N*-[(3-nitro-4-{[(oxan-4-yl)méthyl]amino}phényl)sulfonyl]-2-[(1*H*-pyrrolo[2,3-b]pyridin-5-yl)oxy]benzamide |
| venetoclax | 4-(4-{[2-(4-clorofenil)-4,4-dimetilciclohex-1-en-1-il]metil}piperazin-1-il)-*N*-[(3-nitro-4-{[(oxan-4-il)metil]amino}fenil)sulfonil]-2-[(1*H*-pirrolo[2,3-*b*]piridin-5-il)oxi]benzamida |

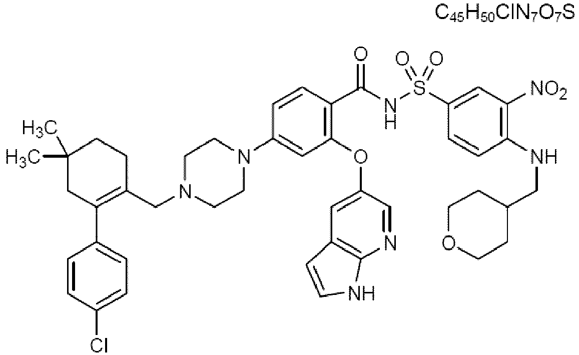

Venetoclax is available under the brand name Venclyxto as 10mg, 50 mg and 100 mg film-coated tablets.

The term "HDM2-p53 interaction inhibitor" or in short "HDM2 inhibitor" is also referred to as "HDM2i", "Hdm2i", "MDM2 inhibitor", "MDM2i", "Mdm2i", denotes herein any compound inhibiting the HDM-2/p53 or HDM-4/p53 interaction with an IC₅₀ of less than 10 µM, preferably less than 1 µM, preferably in the range of nM, measured by a Time Resolved Fluorescence Energy Transfer (TR-FRET) Assay. The inhibition of p53-Hdm2 and p53-Hdm4 interactions is measured by time resolved fluorescence energy transfer (TR-FRET). Fluorescence energy transfer (or Foerster resonance energy transfer) describes an energy transfer between donor and acceptor 5 fluorescent molecules. For this assay, MDM2 protein (amino acids 2-188) and MDM4 protein (amino acids 2-185), tagged with a C-terminal Biotin moiety, are used in combination with a Europium labeled streptavidin (Perkin Elmer, Inc., Waltham, MA, USA) serving as the donor fluorophore. The p53 derived, Cy5 labeled peptide Cy5- TFSDLWKLL (p53 aa18-26) is the energy acceptor. Upon excitation of the donor 10 molecule at 340nm, binding interaction between MDM2 or MDM4 and the p53 peptide induces energy transfer and enhanced response at the acceptor emission wavelength at 665nm. Disruption of the formation of the p53-MDM2 or p53-MDM4 complex due to an inhibitor molecule binding to the p53 binding site of MDM2 or MDM4 results in increased donor emission at 615nm. The ratiometric FRET assay readout is calculated from the 15 raw data of the two distinct fluorescence signals measured in time resolved mode (countrate 665nm/countrate 615nm x 1000). The assay can be performed according to the following procedure: The test is performed in white 1536w microtiterplates (Greiner Bio-One GmbH, Frickenhausen, Germany) in a total volume of 3.1µl by combining 100nl of compounds diluted in 90% DMSO/10% H2O (3.2% final DMSO concentration) with 2µl Europium 20 labeled streptavidin (final concentration 2.5nM) in reaction buffer (PBS, 125mM NaCl, 0.001% Novexin (consists of carbohydrate polymers (Novexin polymers), designed to increase the solubility and stability of proteins; Novexin Ltd., ambridgeshire, United Kingdom), Gelatin 0.01%, 0.2% Pluronic (block copolymer from ethylenoxide and propyleneoxide, BASF, Ludwigshafen, Germany), 1 mM DTT), followed by the addition of 0.5µl MDM2-Bio or MDM4-Bio diluted in assay buffer (final concentration 10nM). Allow the solution to pre-incubate for 15 minutes at room temperature, followed by addition of 0.5µl Cy5-p53 peptide in assay buffer (final concentration 20nM). Incubate at room temperature for 10 minutes prior to reading the plate. For measurement of samples, an Analyst GT multimode microplate reader (Molecular Devices) with the following settings 30 is used: Dichroic mirror 380nm, Excitation 330nm, Emission Donor 615nm and Emission Acceptor 665nm. IC50 values are calculated by curve fitting using XLfit. If not specified, reagents are purchased from Sigma Chemical Co, St. Louis, MO, USA.

The HDM2 inhibitor in accordance with this invention is HDM201, i.e. (S)-5-(5-Chloro-1-methyl-2-oxo-1,2-dihydro-pyridin-3-yl)-6-(4-chloro-phenyl)-2-(2,4-dimethoxy-pyrimidin-5-yl)-1-isopropyl-5,6-dihydro-1H-pyrrolo[3,4-d]imidazol-4-one.

HDM201 may be present as free molecule or in any other non-covalent derivative, including salt, solvate, hydrate, complex, co-crystal or mixtures thereof. HDM201 may be present as acid derivative. The acid derivative may be a salt formed of HDM201 with the acid, or a HDM201 acid complex, or as HDM201 acid co-crystal. Preferably HDM201 is present as co-crystal. Preferably the acid is succinic acid. Most preferably, HDM201 is present as succinic acid co-crystal. Non-covalent derivatives of HDM201 are described in WO2013/111105.

In preferred embodiments, HDM201 is referred to as:
Succinic acid - (6S)-5-(5-chloro-1-methyl-2-oxo-1,2-dihydropyridin-3-yl)-6-(4-chlorophenyl)-2-(2,4 dimethoxypyrimidin-5-yl)-1-isopropyl-5,6-dihydropyrrolo[3,4-d]imidazol-4(1H)-one (1:1).

When referring to a dose amount of HDM201 herein, e.g. in mg (milligram), it is meant to be the amount of HDM201 as free base, in contrast to the salt, solvate, complex, or co-crystal.

The term "hematological tumor" refers herein to a cancer that begins in blood-forming tissue, such as the bone marrow, or in the cells of the immune system. Examples of hematological tumors are leukemia, lymphoma, and multiple myeloma. They are also often referred to as blood cancer.

Preferred hematological tumors of the present invention are leukemias. More preferably, the hematological tumors are selected from acute myeloid leukemia (AML), myelodysplastic syndrome (MDS), and acute lymphoblastic leukemia (ALL). Even more preferably, the hematological tumor is AML and/or MDS.

Particularly preferred hematological tumors of the present invention are *TP53* wild-type hematological tumor. More preferably, the *TP53* wild-type hematological tumors of the present invention are *TP53* wild-type leukemias. Even more preferably, the *TP53* wild-type hematological tumors are selected from TP53 wild-type acute myeloid leukemia (AML), TP53 wild-type myelodysplastic syndrome (MDS), and *TP53* wild-type acute lymphoblastic leukemia (ALL). Even more preferably, the TP53 wild-type hematological tumor is TP53 wild-type AML and/or MDS.

According to the present invention the drug HDM201 is administered on each of the first 3 to 7 days of a 28 days (4 weeks) treatment cycle, preferably the drug is administered on each of the first 4 to 6 days a 28 days treatment cycle, more preferably on the first 5 days of a 28 days treatment cycle.

"On each of the the first 5 days of a 28 days treatment cycle" means that HDM201 is administered to the patient on day 1 (d1), d2, d3, d4, and d5, followed by a drug-administration-free period (also referred to as drug holiday period or rest period) from day 6 until day 28. On day 29 the next treatment cycle starts which will be the d1 of this next treatment cycle.

Preferably, the drug is administered at approximately the same time each administration day (i.e. d1-d5 of a 28 days cycle). Preferably, the drug is administered once daily (qd) on each administration day. More preferably, the drug is administered in the morning.

Preferably, the drug is administered in the fasted state, i.e. at least 1 hour before or 2 hours after a meal.

Preferably the drug is taken with a glass of water and without chewing the capsules or tablet.

If the patient is assigned to a dose level where multiple capsules/tablets are to be taken, the capsules/tablets should be taken consecutively, within as short an interval as possible, e.g. within 5 min.

Preferably, the drug administration is done by oral delivery, i.e. oral administration, per oral (p.o.).

Preferably the drug is provided in the form of an oral dosage form, more preferably in the form of a solid oral dosage form, e.g. a capsule or a tablet.

When dose ranges are given herein, e.g. "the daily drug dose is from 50 mg to 100 mg", any full mg number of the endpoints and in the between those endpoint shall be meant to be disclosed herewith, e.g. 50 mg, 51 mg, 52 mg, 53 mg, 54 mg, 55 mg, 56 mg, 57 mg, ... 98 mg, 99 mg, 100 mg.

As a further aspect of the present invention there is provided:
The combination of HDM201 and venetoclax in accordance with any one of the embodiments as described herein, wherein said combination is combined with one or more other/further anti-cancer agents, preferably said anti-cancer agent(s) is(are) selected from: immuno-oncological drugs (e.g. PD-1 [e.g. PDR001 (Novartis, INN Spartalizumab)], PD-L1, LAG-3, TIM-3 [e.g. MBG453(Novartis)], GTIR, TGF-beta, IL15 inhibitors), FLT3 inhibitors (e.g. gilterinib, quizartinib, midostaurin), BCL2 inhibitors (e.g. navitoclax, venetoclax), other HDM2 inhibitors (e.g. idasanutlin, AMG232, DS-3032B, ALRN6924/ATSP7041), hypomethylating agents (HMA) (e.g. Vidaza [azacytidine, 5-azacytidine], Dacogen [decitabine], guadecitabine), anthracyclines (e.g. idarubicin, daunorubicin, doxorubicin, epirubicin, rubidomycin); anti-CD33 antibodies (e.g. Mylotarg [gemtuzumab], vadastuximab) and other agents (e.g. AraC [cytarabine, aracytine]).

Preferably, the combination of HDM201 and venetoclax is combined with one or more therapeutically active agents selected from cytarabine (Ara-C), anthracycline, daunorubicin, idarubicin, rubidomycin, idamycin, midostaurin and azacytidine.

In other particular preferred embodiments, the combination of HDM201 and venetoclax is combined with a TIM-3 inhibitor, preferably the TIM-3 inhibitor MBG453 (Novartis). TIM-3 inhibitors, e.g. MBG453, are described in WO 2015/117002, the entire content of which is incorporated herein by reference.

The other/further active agents may be dosed on the same day(s) as HDM201 or on days on which no HDM201 dose is administered.

The second medical uses as described in the embodiments of the present invention may be worded in the following various alternative formats:
The combination of HDM201 and venetoclax for use in the treatment of cancer.

A method for the treatment of cancer in human patients in need of such treatment which comprises administering an effective amount of the combination of HDM201 and venetoclax.

Use of the combination of HDM201 and venetoclax for the manufacture/preparation of a medicament for the treatment of cancer.

A medicament for the treatment of cancer comprising the combination of HDM201 and venetoclax.

### EXAMPLES

### Example 1: HDM201 dosing regimen modeling

### Platelet model

Based on the population PK/PD data of the clinical study CHDM201X2101, an AML patients platelet model was developed which recognizes that the disease influences the regulation of platelets production. The following graphic elucidates the model.

At time 0, B(0) + PLT(0) = PLTN=270 G/L
PLB(t)
E'([C])*exp(-lba*t)
Regulation = (PLTN / (PLT(t)+PLB(t)))^γ
PLT Transfusion
MMT
Prol.
A2
A3
A4
Circ.
E([C])

### Bone marrow blasts model

A bone marrow blasts PKPD model were developed which recognizes a delayed effect, a loss of effect with time reproduced by a resistance component, and that a concentrated administration reduces impact of resistance. The following graphic elucidates the model.

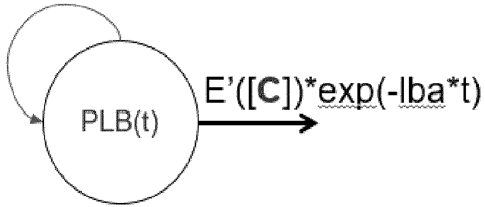

### Derivation of key metrics from simulated platelet and blast profiles

The population PK/PD models of example 1 and 2 were used to simulate PK, platelet and blast profiles over time with inter-individual variability.

The impact of a change in dosing regimen on these profiles were studied.

The simulation design considered: Duration of the cycle, Dose level, Number of administration, Duration of treatment, Period of induction/consolidation.

The key metrics were: Proportion of patients with platelet counts below/above a given threshold over time, Proportion of patients above PK threshold, Number of days with Blast values below baseline.

The simulations were done using the R (statistical software) with Shiny package.

For model building the PK/PD dataset of CHDM201X2101 were used and an NLME estimation (Monolix 4.3.2) performed. The model structure and the parameter estimates are provided below. This provided inputs for R/shiny. The *mlxR* package were used for simulation of longitudinal data from the MLXTRAN model.

Model structure

INPUT:
parameter = {r, t1, t2, Tk0, ka, V, Cl, PLTz, MMTP, T12P, sPW, alp, lPW,
kr1, kr1D, EC50, ke0,cfx,h, Sg, HGD, koutg, gdfZ, kinG}

PK:
compartment(cmt=1, amount=Ac)
compartment(cmt=2, amount=P5)
absorption(adm=1, Tlag=t1, Tk0, p=r)
absorption(adm=1, Tlag=t2, ka, p=1-r)
TinfP = 0.5; infusion duration in hours
oral(adm=2, cmt=2, Tk0=TinfP, p=alp)

EQUATION:
odeType = stiff
C = max(1e-16,Ac/V)*1000; convert to ng/mL the concentrations
Cc = C
ke =Cl/V
ddt_Ac = - ke*Ac

ktrP = 4/MMTP
KTR12 = log(2)/T12P
auxF = PLTz/ktrP*KTR12

sfbkP = (PLTz/P5)^(sPW*exp(cfx*E))
lfbkP = (auxF/P1)^lPW

EP1 = kr1*E^h/(E^h+EC50^h) + kr1D*Cc

P1_0 = auxF
P2_0 = auxF
P3_0 = auxF
P4_0 = auxF
P5_0 = PLTz

ddt_P1 = ktrP*(sfbkP-EP1)*P1- ktrP*P1
ddt_P2 = ktrP*lfbkP*sfbkP *P1 - ktrP*P2 ;- EP2*P2
ddt_P3 = ktrP*lfbkP*sfbkP *P2 - ktrP*P3 ;- EP3*P3
ddt_P4 = ktrP*lfbkP*sfbkP *P3 - ktrP*P4 ;- EP4*P4
ddt_P5 = ktrP*lfbkP*sfbkP *P4 - KTR12*P5

ddt_E = ke0*Cc - ke0*E

Parameter estimates

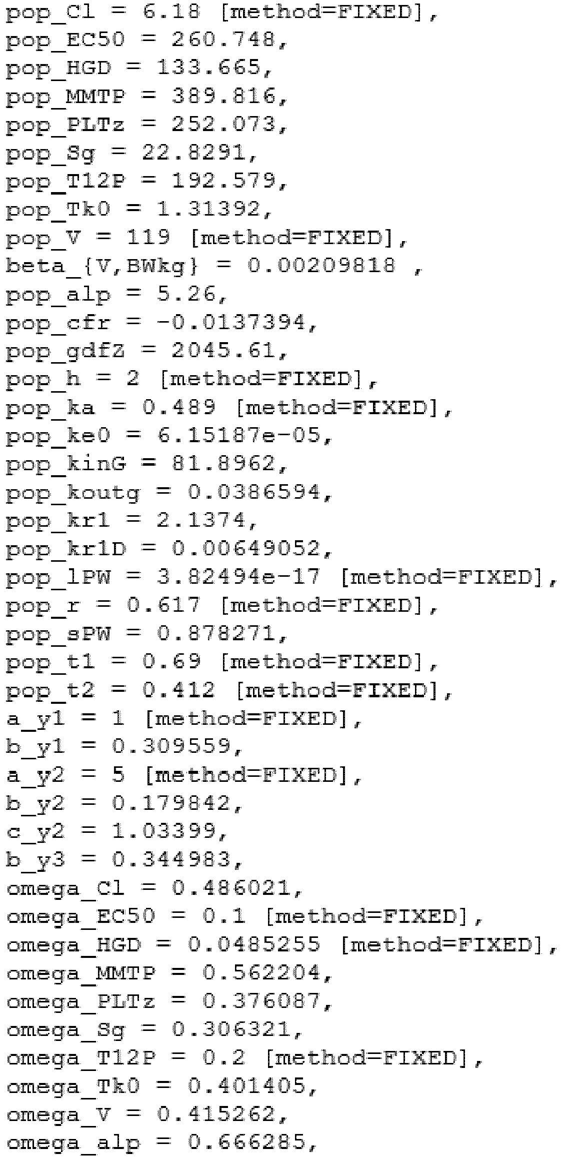

As key findings from PKPD simulations the following was found:
- Long-term platelet depletion and
- Long term treatment (>6 months) is not sustainable without a dose reduction or interruption:
   - Progressive reduction of platelet counts with increasing treatment cycles
   - Disease resistance limiting drug effect on blasts beyond cycle 3 or 4

The simulations support dose and regimen selection for Phase 2 studies in AML.

As a learning from the clinical study CHDM201X2101, the challenges with dosing HDM210 in AML are
- Cumulative platelet toxicity
- Delayed hematopoietic recovery that prevents dosing in consolidation would present a risk to this indication

The present simulation provides a good management of those challenges:
Dose reduction after 1 or 2, preferably 2 cycles of induction.

The simulation was used to support dose escalation strategy in the clinical study HDM201A2101: a new D1-D5 (4 wk cycle) regimen instead of regimen D1-D7 (4 wk cycle) was identified. The following table provides the details of the new dose escalation and new dose regimens.

**Table 1: Simulation of platelet (PLT) and bone marrow (BM) blast metrics from HDM201X2101**

| **Cohort** | Dose Regimen for HDM201 induction Cycles 1+2 | Dose Regimen for HDM201 consolidation Cycles 3-5 | Median % subjects above target [C] from Cycle 1¹ | Median % subjects with at least 1 PLT value above threshold 50G/L² | No. of days with BM blast value below baseline^{2,3} | Median % subjects with PLT decrease from baseline ≥ 50%² | Median % subjects with PLT decrease from baseline ≥ 75%² |
|---|---|---|---|---|---|---|---|
| **-1A** | 60 mg, D1 | 60 mg, D1 | 3 [2.8-3.4] | 3.2 [2.4-3.4] | 7.8 [7.1-8.3] | 29 [27.8-29.8] | 12.6[12.4-13.2] |
| **-1B** | 45 mg, D1-D2 | 45mg, D1-D2 | 15.2 [14-16.2] | 7.8 [7.4-8.2] | 12.1 [11.4-13] | 39.8 [38.8-40.7] | 20.8 [20.4-21.4] |
| **Starting 1** | 40 mg, D1-D3 | 40 mg, D1-D3 | 35 [34-35.6] | 12.2 [11.8-12.6] | 16.8 [15.6-17.3] | 48.6 [48-49.3] | 29.2 [27.9-29.6] |
| **2** | 40 mg, D1-D5 | 40 mg, D1-D5 | 69.4 [69-69.9] | 19.4 [19-19.8] | 38.2 [33.9-41.8] | 63.2 [62.8-63.6] | 41 [40.2-41.6] |
| **3** | 60 mg, D1-D5 | 40 mg, D1-D5 | 89.6 [88.9-90] | 25.2 [24.6-25.8] | 63.1 [58.8-65.9] | 69.9[69.2-70.4] | 48.4 [47.4-49.1] |
| **4** | 80 mg, D1-D5 | 40 mg, D1-D5 | 96.8 [96.4-97.2] | 29.4 [28.2-30] | 82 [77.8-86.2] | 76.4 [74.9-78.8] | 57.4 [56.1-59.7] |
| Note: Metric values represent the Median (2.5%-97.5% percentiles) of 100 repeated simulations performed on 500 subjects. | | | | | | | |
| ¹ average tumor stasis concentration derived from tumor growth inhibition (PK/PD) modeling in xenograft rat model. | | | | | | | |
| ² metric value calculated from Day 1 to Day 140. | | | | | | | |
| ³ subjects with no observed blast reduction from baseline were excluded from the metric derivation. | | | | | | | |

### Example 2: Pre-clinical study

### Example 2: In vivo pharmacology of HDM201 and Venetoclax combination

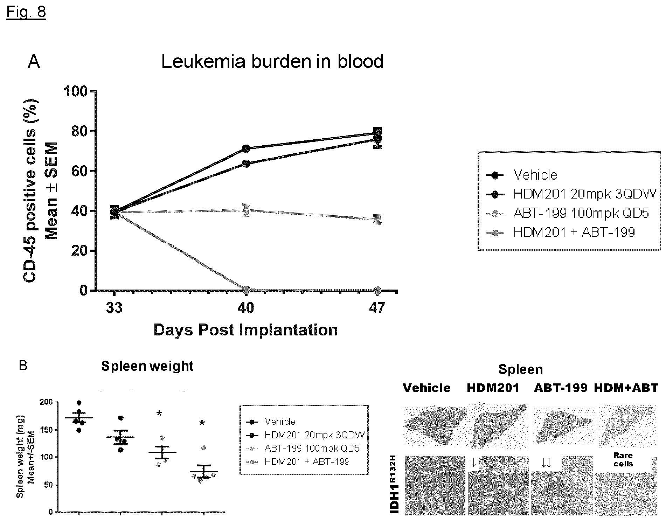

HDM201 was shown to enhance antitumor activity of a selective Bcl-2 inhibitor venetoclax *in vivo* in multiple AML patient derived orthotopic models. In mice harboring the mutant *IDH1*/*FLT3-ITD,* HDM201 treatment alone exhibited minimal anti-cancer activity (92%T/C, p>0.05). In contrast, HDM201 in combination with venetoclax induced complete tumor regressions (-100%Reg), while only partial tumor regressions were observed with venetoclax alone (-9 to -52%Reg). See Figures 8 and 9.

Consistent with the observation in peripheral blood, the depletion of leukemic cells in spleen was also observed by spleen weight and IHC staining of IDH1^{R132H} positive leukemic cells. HDM201 as a single agent led to a modest reduction of spleen size and leukemic density. In contrast, HDM201 in combination with Venetoclax resulted in a near complete depletion of the leukemic cells in spleen and significant reduction of spleen size comparable to naïve animals, while Venetoclax alone exhibited partial reduction of spleen size and leukemic density as compared to the vehicle control (See Figure 8).

In an additional study, HDM201 was tested at three dose levels (5, 10, and 20 mg/kg HDM201) in combination with venetoclax. HDM201 given at a 4 fold lower dose (5 mg/kg vs 20 mg/kg) in venetoclax combination, improved the platelet counts to the levels comparable to non-treated animals, while still maintaining the high degree of tumor regression (-75%Reg) (see Figure 9). These data suggest that potential overlapping hematological toxicities of the combination could be mitigated with administration of lower doses of HDM201 while maintaining the anti-tumor activity.

### Example 3: Clinical study

### Rationale and design for dose/regimen and duration of treatment of HDM201 in combination with venetoclax

This is a phase 1b, multi-arm, open-label study of HDM201 in combination with venetoclax in subjects with AML or high-risk MDS.

For all subjects, TP53wt status must be characterized by, at a minimum, no mutations noted in exons 5, 6, 7 and 8.

Subjects will receive HDM201 in combination with venetoclax.

Venetoclax dose will be gradually increased (ramp-up) over a period of 4 to 5 days to achieve the target daily dose to be tested (either 400 or 600 mg) as shown in the following graphic:

Ramp-up (RU) of venetoclax during cycle 1 (treatment arm 2: HDM201+venetoclax)

Day 3
Day 4-28
Day 2
200 mg
400 mg
Day 1
100 mg
Day 5-28
Primary
ramp-up
(over 4 days)
50 mg
Day 4
600 mg
Day 3
400 mg
Day 2
200 mg
Day 1
100 mg
Alternative
ramp-up
(over 5 days)
50 mg

Once subjects receive the planned target daily dose, they will continue at that dose of venetoclax.

The HDM201 dose may be escalated (see Table 3-1 for provisional dose levels to be tested). Based on the potential for cumulative HDM201-related safety effects with repeat dosing, subjects will not receive an HDM201 dose greater than the planned highest dose of 40 mg daily (>200 mg/cycle) from cycle 3 onwards.

Upon the completion of the escalation part, MTD(s) and/or RD(s) of HDM201 in combination with venetoclax in AML and high-risk MDS subjects will be determined.

Study treatment will be administered in 28-day dosing cycles.

Each treatment arm will enroll cohorts of 3 to 6 subjects treated with HDM201+venetoclax until MTD(s) and/or RD(s) and regimen for future use are identified.

Additional cohorts of 1 to 10 subjects may be enrolled at a previously tested and declared safe dose level in one or both indications in order to better understand the safety, tolerability, PK and preliminary activity of study treatments.

This is the first trial that will evaluate the combination of HDM201 with venetoclax. A pharmacokinetic drug-drug interaction is possible and the combination of HDM201 with venetoclax may have overlapping toxicities.

In this study, the selection of the dose and regimen for HDM201 is based on the currently available preclinical and clinical safety, efficacy, PK and PKPD modeling information from the single agent first-in-human clinical trial CHDM201X2101, while for venetoclax the dose and regimen are selected from clinical data available for venetoclax trials in AML (Konopleva et al. 2016 and DiNardo et al. 2018).

A dose-escalation approach will be undertaken in order to determine the appropriate dose of HDM201 in combination with venetoclax. The starting dose of HDM201 tested in combination with venetoclax will be 20 mg. HDM201 will be administered orally once daily from day 1 to day 5 of a 28 days cycle. Based on PBPK SimCyp modeling, at the 20 mg HDM201 starting dose (daily from day 1 to 5, 28-day cycle) in combination with venetoclax (continuous daily) a minimal DDI effect is anticipated with a predicted -1.20-fold increase of venetoclax exposure (AUC) during the initial ramp-up phase and at steady state. Under the planned combination dosing for HDM201 (40 or 60 mg daily from day 1 to 5, 28-day cycle) and venetoclax (continuous daily), exposure (AUC and Cmax) to venetoclax was predicted to increase by <2-fold. The PK of venetoclax, however, is predicted to gradually return to expected levels within 3-5 days after the fifth and last administration of HDM201. This transient and limited increase in the exposure of venetoclax is unlikely to necessitate a venetoclax dose adjustment during the period of coadministration with HDM201. Furthermore, venetoclax was administered up to 1200 mg daily without reaching the maximal tolerated dose. Thus, even if an increase of venetoclax exposure is expected during the coadministration period with HDM201, the anticipated impact is low.

To mitigate the risk of TLS and DDI impact, venetoclax will be administered orally once daily continuously and will be gradually increased during the first treatment cycle with a 4 to 5 days ramp-up (RU) period until the target daily dose of venetoclax per cohort is reached. Subsequently, subjects will continue at this target daily dose of venetoclax.

Regarding the target dose of venetoclax, a phase 1b study ([NCT02203773]) has treated overall 10 AML subjects with venetoclax 400 mg daily in combination with hypomethylating agents with a tolerable safety profile, and the expansion cohorts are now ongoing at 400 mg (daily) and a higher dose (800 mg with an interrupted dosing schedule) in combination (DiNardo et al. 2018). In addition, several ongoing phase 2 and phase 3 studies conducted in R/R, unfit for chemotherapy AML, and high-risk MDS subjects, are testing venetoclax in combination with standard of care (low-dose chemotherapy or hypomethylating agents), using either venetoclax at a target dose of 400 mg or 600 mg ([NCT03404193], [NCT02993523], [NCT03069352]). Based on the above, the daily target dose of venetoclax in the study has been chosen to be either 400 mg or 600 mg daily.

Several studies testing venetoclax either as single agent or in combination in R/R, unfit for chemotherapy AML, and MDS subjects and using a similar ramp-up (i.e. 3 to 5 days) have reported a very low (<5%) incidence of TLS ([NCT01994837], [NCT02670044], [NCT03214562], [NCT02287233], [NCT02993523]). The aim of the ramp-up is to gradually reduce the tumor burden to mitigate the risk of TLS, especially in the context of a combination of investigational drugs expected to be synergistic.

Several studies testing venetoclax either as a single agent or in combination in AML and MDS have reported different starting doses, either 20 mg, 50 mg or 100 mg daily. In the phase 2 study testing venetoclax as a single agent, the starting dose was 20 mg daily with a ramp-up over 5 days, and no TLS has been observed ([NCT01994837]). In the phase 1b study ([NCT02203773]) testing venetoclax with hypomethylating agents, of the 57 enrolled subjects, only 10 started at 20 mg of venetoclax, while the others started either at a higher dose (50 mg or 100 mg daily) with a ramp-up over 5 days and no TLS has been reported (DiNardo CD, Pratz KW, Letai A, et al (2018) Safety and preliminary efficacy of venetoclax with decitabine or azacitidine in elderly patients with previously untreated acute myeloid leukaemia: a non-randomised, open-label, phase 1b study. Lancet Oncol; 19(2):216-228). Another phase 1b/2 study testing venetoclax in combination with LDAC ([NCT02287233]) has used 50 mg as the starting dose with a ramp-up over 5 days with no case of clinically significant TLS being reported (Lin TL, Strickland SA, Fiedler W, et al (2016) Phase Ib/2 study of venetoclax with low-dose cytarabine in treatment-naive patients age ≥ 65 with acute myelogenous leukemia. J Clin Oncol; 34(15):suppl 7007-7007). Based on the above, the starting dose of venetoclax will be 50 mg daily and the ramp-up primarily explored will be over 4 days with a daily target dose of venetoclax of 400 mg. Alternatively, a ramp-up over 5 days with a daily target dose of venetoclax of 600 mg may be explored based on the observed clinical safety and PK data, according to the BHLRM. In this arm both HDM201 and venetoclax may be dose escalated; however only one of the two investigational drugs (i.e. HDM201 or venetoclax) will be escalated at a time. The escalation of each of the two study drugs may be tested in parallel in two different cohorts with a reduced cohort size, if both escalations are allowed by the BHLRM (i.e. the corresponding dose combinations are admissible).

Based on these prior safety data and the assumptions for DDI, the starting dose for the combination satisfies the EWOC criteria within the BHLRM.

### Rationale for choice of combination drugs

The rationale for combining HDM201 and venetoclax is based on the following evidence:
- Preclinical evidence shows that the concurrent blockade of MDM2 and Bcl-2 has synergistic activity in p53wt AML subject samples and exerts synergistic anti-tumor activity in different AML xenograft mouse models (Example 2).
- Early clinical data of the MDM2 inhibitor idasanutlin in combination with venetoclax report preliminary activity in AML subjects (Daver N, Pollyea DA, Yee K, et al (2017) Preliminary Results from a Phase Ib Study Evaluating BCL-2 Inhibitor Venetoclax in Combination with MEK Inhibitor Cobimetinib or MDM2 Inhibitor Idasanutlin in Patients with Relapsed or Refractory (R/R) AML. The American Society of Hematology -59th Annual Meeting and Exposition, Atlanta, Georgia).

### Population

The study is conducted in TP53wt adult patients with:
- R/R AML who have failed ≥1 prior regimen, or
- First line AML unfit for standard induction chemotherapy, or
- High-risk MDS who have failed hypomethylating agent therapy.

Only patients who meet all the following inclusion and none of the exclusion criteria are treated in the study. National Cancer Institute CTCAE version 5.0 is used for all grading.

### Inclusion criteria

Patients eligible for inclusion in this study must meet all of the following criteria:
1. Male or female patients ≥ 18 years of age at the date of signing the informed consent form who present with one of the following:
   a. Relapsed/refractory AML following ≥1 prior therapies (but ≤3 prior therapies) who have relapsed or exhibited refractory disease (primary failure) and are deemed by the Investigator not to be candidates for standard therapy, including re-induction with cytarabine or other established chemotherapy regimens for patients with AML (patients who are suitable for standard re-induction chemotherapy or hematopoietic stem cell transplantation and willing to receive it are excluded). In an embodiment, the AML is Relapsed/refractory AML following one or more prior therapies, in patients who have relapsed or exhibited refractory disease (primary failure).
   b. First line AML patient unfit for standard induction chemotherapy (includes both de novo and secondary AML). Patients who are suitable for hematopoietic stem cell transplantation and willing to receive it are excluded. In another embodiment, the AML is First line AML, particularly in patient(s) unfit for standard induction chemotherapy, (wherein the AML includes both de novo and secondary AML).
   c. High-risk MDS patient (high and very high-risk groups according to rIPSS) who have failed hypomethylating agent therapy. In another embodiment, the MDS is High-risk MDS patient (high and very high-risk groups according to rIPSS), in particular, patients who have failed hypomethylating agent therapy.2. Eastern Cooperative Oncology Group (ECOG) performance status ≤ 1
3. Tumor of the patient is TP53wt . At minimum exons 5, 6, 7 and 8 in the TP53 gene must be sequenced and determined to contain no mutations. The TP53 status must be obtained from a bone-marrow sample, collected no longer than 3 months before signing the main ICF.
4. Patients are candidates for serial bone marrow aspirate and/or biopsy according to the institutions guidelines and undergo a bone marrow aspirate and/or biopsy at screening, during and at the end of therapy on this study.

### Principle exclusion criteria:

Patients eligible for this study must not meet any of the following criteria:
- Prior combination treatment with compounds having the same mode of action:
   - mdm2 or mdm4 inhibitors combined with Bcl-2 inhibitor
- History of severe hypersensitivity reactions to any ingredient of study drug(s) and other monoclonal antibodies (mAbs) and/or their excipients.
- Patients with acute promyelocytic leukemia with PML-RARA.
- Allogeneic stem cell transplant (HSCT) within last 6 months and/or active GvHD requiring systemic immunosuppressive therapy.
- GI disorders impacting absorption of oral HDM201 or venetoclax.
- Evidence of active bleeding or bleeding diathesis or major coagulopathy (including familial).

### Treatment and study drugs

For this study, the term "investigational drug" or "study drug" refers to HDM201 or venetoclax. "Treatment arm" or "study treatment" refers to a specific combination treatment i.e. HDM201+venetoclax. The investigational drugs used in this study are:
HDM201, 10mg, 20mg, 40mg, Capsule for oral use, 20 mg (starting dose), Day 1 to day 5 (28-day cycle), Open label patient specific; bottles.

Venetoclax, Tablet for oral use, 400 mg OR 600 mg, 4 to 5 days ramp up + daily (28-day cycle), Open label patient specific; as available locally.

No randomization will be performed in this study.

HDM201 capsules will be administered orally (p.o.) in the fasted state at least 1 hour before or 2 hours after a meal. The subject should take the capsules in the morning, at approximately the same time each day of dosing, with a glass of water and without chewing the capsules. If the subject is assigned to a dose level where multiple capsules are to be taken, the capsules should be taken consecutively, within as short an interval as possible. On the visit days, the subject will take HDM201 at the clinic under the supervision of the Investigator or designee and on other days at home. If the subject forgets to take his/her daily dose, then he/she should restart the dose on the next scheduled dosing day without compensating for missed doses. Any missed study medication should be reported to the Investigator at the next study visit. HDM201 is to be administered first.

Venetoclax film-coated tablets will be administered orally once a day according to local label recommendations. Subjects should be instructed to swallow the tablets as a whole with water at approximately the same time each day. The tablets should be taken with a meal (ideally during breakfast) in order to avoid a risk for lack of efficacy.

During the ramp-up period, venetoclax should be taken in the morning to facilitate laboratory monitoring.

If a subject misses a dose of venetoclax within 8 hours of the time it is usually taken, the subject should take the missed dose as soon as possible on the same day. If a subject misses a dose by more than 8 hours, the subject should not take the missed dose and should resume the usual dosing schedule at the usual time the following day. However, in case the missed dose occurs during the ramp-up period of venetoclax, the subject should resume on the following day at the dose that was missed in order not to skip any dose increase of venetoclax during the ramp-up.

On the days of PK sampling, the subject will be instructed to bring his/her drug supply to the site, and take the dose in the clinic, under supervision of the site personnel.

A subject may continue study treatment until the subject experiences unacceptable toxicity, disease progression (Cheson BD, Bennett JM, Kopecky K, et al (2003) Revised recommendations of the International Working Group (IWG) for diagnosis, standardization of response criteria, treatment outcomes, and re orting standards for therapeutic trials in acute myeloid leukemia. J Clin Oncol; 21(24):4642-9 and Cheson BD, Greenberg P, Bennett J, et al (2006) Clinical application and proposal for modification of the International Working Group (OWG) response criteria in myelodysplasia. Blood; 108:419-425). If more than 2 consecutive cycles of HDM201+venetoclax have to be skipped due to drug-related toxicities, then the combination of drugs should be permanently discontinued.

### Dose escalation and dose modification

### Starting dose

The starting dose and regimen selection for HDM201 in dose escalation is based on the previous Phase I dose escalation and expansion study of HDM201 as a single-agent in subjects with AML/MDS (CHDM201X2101) in which a dose of 45 mg/day (day 1-7 / 28-day cycle) was determined to be the RD. In this study, a starting dose and regimen of 20 mg/day HDM201 (day 1-5 / 28-day cycle) for dose escalation has been selected. The selection of dose and regimen was supported by single agent translational preclinical modeling of tumor bearing rats and population PK/PD modeling of thrombocytopenia and bone marrow blast data from CHDM201X2101 study in AML/MDS subjects. The starting dose corresponds to -315% below the cumulative dose of HDM201 single agent RD (as evaluated in CHDM201X2101 at 45 mg/day (day 1-7 / 28-day cycle), or 315 mg/cycle). At this dose level, -15% of subjects are predicted to achieve preclinical derived average target efficacious concentrations of HDM201 per cycle, with some anticipated clinical activity (bone marrow blast reduction) and limited target myelosuppression. Additionally, PBPK SimCyp modeling at the 20 mg HDM201 starting dose predicted -1.20-fold increase of venetoclax exposure during the initial ramp-up phase and at steady state.

In the HDM201+venetoclax treatment arm 2, the starting dose for HDM201 is 20 mg/day (day 1-5 / 28-day cycle). The venetoclax starting dose of 50 mg will be gradually increased over a period of 4 days in order to reach the planned target dose of 400 mg/day of venetoclax. Once this target dose is reached, subjects will continue on that dose of venetoclax (28-day cycle). Only one of the two investigational drugs (i.e. HDM201 or venetoclax) can be escalated at a time. However, the escalation of each of the two study drugs may be tested in parallel in two different cohorts but with a reduced cohort size, if both escalations are allowed by the BHLRM (i.e. the corresponding dose combinations are admissible).

### Provisional dose levels

The following Table 3-1 describes the starting dose and the dose regimen of HDM201 that may be evaluated during the combination HDM201+venetoclax. (1 cycle = 28 days).

| Dose level | HDM201 dose, cycles 1-2* | HDM201 dose, cycles ≥3* |
|---|---|---|
| -1 ** | 10 mg, d1-5 | 10 mg, d1-5 |
| 1 (start) | 20 mg, d1-5 | 20 mg, d1-5 |
| 2 | 30 mg, d1-5 | 30 mg, d1-5 |
| 3 | 40 mg, d1-5 | 40 mg, d1-5 |
| 4 | 50 mg, d1-5 | 40 mg, d1-5 |
| 5 | 60 mg, d1-5 | 40 mg, d1-5 |

| | | |
|---|---|---|
| * It is possible for additional and/or intermediate dose levels to be added during the course of the study. Cohorts may be added at any dose level below the MTD in order to better characterize safety, PK or PD. ** Dose level -1 represents treatment dose when dose de-escalation from the starting dose level is required. No dose de-escalation below dose level -1 is permitted for this study. | | |

The following Table describes the starting dose and ramp-up of venetoclax that may be evaluated during the HDM201+venetoclax combination (treatment arm 2). Refer to paragraphs above for details on the dosing during the ramp-up period.

| Dose level | Venetoclax daily dose | Venetoclax dosing frequency |
|---|---|---|
| -1* | 300 mg | |
| 1 (start) | 400 mg | Ramp-up 4 days, then daily |
| 2 | 600 mg | Ramp-up 5 days, then daily |

| | | |
|---|---|---|
| * Dose level -1 represents treatment dose when dose de-escalation from the starting dose level is required. No dose de-escalation below dose level -1 is permitted for this study. | | |

### Objectives and endpoints

| Objectives | Endpoints | |
|---|---|---|
| Primary Objective(s): | Endpoint(s) for primary objective(s) | |
| To characterize safety and tolerability of each treatment arm and identify recommended doses and regimens for | Safety: | |
| | - Incidence and severity of AEs and SAEs, including changes in laboratory | |
| future studies | values, vital signs, and ECGs. | |
| | - Incidence and nature of DLTs. | |
| | Tolerability: Dose interruptions, reductions, and dose intensity | |
| Secondary Objective(s): | Endpoint(s) for secondary objective(s): | |
| To characterize the pharmacokinetic profile of investigational drugs (HDM201, and venetoclax) administered in combination. | PK parameters (e.g., AUC, Cmax, Tmax) and concentration vs. time profiles of each investigational drug within combination regimens. | |
| To evaluate preliminary anti-tumor activity. | | |
| To assess the pharmacodynamics (PD) effect. | ORR, BOR and: | |
| | | - EFS, RFS and DOR for AML (Cheson 2003) |
| | | - PFS, TTR and DOR for MDS (Cheson 2006) Changes from baseline in GDF-15 |

### List of abbreviations:

- AE: Adverse Event
- SAE: Serious Adverse Event
- AUC: Area Under the Curve
- AML: Acute Myeloid Leukemia
- R/R: Relapsed/Refractory
- BHLRM: Bayesian Hierarchical Logistic Regression Model
- BM: Bone Marrow
- CR: Complete Remission
- CTCAE: Common Terminology Criteria for Adverse Events
- MDS: Myelodysplastic Syndrome
- MTD: Maximum Tolerated Dose
- RD: Recommended Dose
- FIH: First in Human
- EWOC: Escalation with Overdose Control
- Q4W: Every 4 weeks
- Q2W: Every 2 weeks
- TP53: Tumor Protein 53
- Wt: wild type
- PML-RARA: Promyelocytic leukemia/retinoic acid receptor alpha
- GvHD: Graft versus host disease
- G!: Gastrointestinal
- ECG: Electrocardiogram
- DLT: Dose Limiting Toxicity
- ORR: Overall Response Rate
- BOR: Best Overall Response
- PFS: Progression Free Survival
- TTR: Time To Response
- DOR: Duration of Response
- rIPSS: revised International Prognostic Scoring System
- DDI: Drug-Drug Interaction
- ICF: Informed Consent Form

## Claims

1. The HDM2-p53 interaction inhibitor drug (S)-5-(5-Chloro-1-methyl-2-oxo-1,2-dihydro-pyridin-3-yl)-6-(4-chloro-phenyl)-2-(2,4-dimethoxy-pyrimidin-5-yl)-1-isopropyl-5,6-dihydro-1H-pyrrolo[3,4-d]imidazol-4-one [HDM201] or a pharmaceutically acceptable non-covalent derivative (including salt, solvate, hydrate, complex, co-crystal) thereof for use in the treatment of cancer, wherein the treatment further comprises administration of the BCL2 inhibitor 4-(4-{[2-(4-chlorophenyl)-4,4-dimethylcyclohex-1-en-1-yl]methyl}piperazin-1-yl)-N-[(3-nitro-4-{[(oxan-4yl)methyl]amino}phenyl)sulfonyl]-2-[(1H-pyrrolo[2,3-b]pyridin-5-yl)oxy]benzamide [venetoclax], or a pharmaceutically acceptable non-covalent derivative (including salt, solvate, hydrate, complex, co-crystal) thereof, and a hypomethylating agent.

2. The BCL2 inhibitor 4-(4-{[2-(4-chlorophenyl)-4,4-dimethylcyclohex-1-en-1-yl]methyl}piperazin-1-yl)-N-[(3-nitro-4-{[(oxan-4yl)methyl]amino}phenyl)sulfonyl]-2-[(1H-pyrrolo[2,3-b]pyridin-5-yl)oxy]benzamide [venetoclax], or a pharmaceutically acceptable non-covalent derivative (including salt, solvate, hydrate, complex, co-crystal) thereof for use in the treatment of cancer, wherein the treatment further comprises administration of the HDM2-p53 interaction inhibitor drug (S)-5-(5-Chloro-1-methyl-2-oxo-1,2-dihydro-pyridin-3-yl)-6-(4-chloro-phenyl)-2-(2,4-dimethoxy-pyrimidin-5-yl)-1-isopropyl-5,6-dihydro-1H-pyrrolo[3,4-d]imidazol-4-one [HDM201] or a pharmaceutically acceptable non-covalent derivative (including salt, solvate, hydrate, complex, co-crystal) thereof, and a hypomethylating agent.

3. A hypomethylating agent for use in the treatment of cancer, wherein the treatment further comprises administration of the HDM2-p53 interaction inhibitor drug (S)-5-(5-Chloro-1-methyl-2-oxo-1,2-dihydro-pyridin-3-yl)-6-(4-chloro-phenyl)-2-(2,4-dimethoxy-pyrimidin-5-yl)-1-isopropyl-5,6-dihydro-1H-pyrrolo[3,4-d]imidazol-4-one [HDM201] or a pharmaceutically acceptable non-covalent derivative (including salt, solvate, hydrate, complex, co-crystal) thereof, and the BCL2 inhibitor 4-(4-{[2-(4-chlorophenyl)-4,4-dimethylcyclohex-1-en-1-yl]methyl}piperazin-1-yl)-N-[(3-nitro-4-{[(oxan-4yl)methyl]amino}phenyl)sulfonyl]-2-[(1H-pyrrolo[2,3-b]pyridin-5-yl)oxy]benzamide [venetoclax], or a pharmaceutically acceptable non-covalent derivative (including salt, solvate, hydrate, complex, co-crystal) thereof.

4. The HDM2-p53 interaction inhibitor drug for use according to claim 1, the BCL2 inhibitor for use according to claim 2, or the hypomethylating agent for use according to claim 3, wherein the cancer is a hematological tumor.

5. The HDM2-p53 interaction inhibitor drug for use according to claim 1 or claim 4, the BCL2 inhibitor for use according to claim 2 or claim 4, or the hypomethylating agent for use according to claim 3 or claim 4, wherein the hypomethylating agent is azacitidine.

6. The HDM2-p53 interaction inhibitor drug for use according to any one of claims 1, 4 and 5, the BCL2 inhibitor for use according to any one of claims 2, 4 and 5, or the hypomethylating agent for use according to any one of claims 3 to 5, wherein the hematological tumor is acute myeloid leukemia (AML).

7. The HDM2-p53 interaction inhibitor drug for use according to claim 6, the BCL2 inhibitor for use according to claim 6, or the hypomethylating agent for use according to claim 6, wherein the AML is relapsed/refractory AML.

8. The HDM2-p53 interaction inhibitor drug for use according to any one of claims 1 and 4 to 7, the BCL2 inhibitor for use according to any one of claims 2 and 4 to 7, or the hypomethylating agent for use according to any one of claims 3 to 7, wherein the cancer is a TP53 wild-type tumor.

9. The HDM2-p53 interaction inhibitor drug for use according to any one of claims 1 and 4 to 8, the BCL2 inhibitor for use according to any one of claims 2 and 4 to 8, or the hypomethylating agent for use according to any one of claims 3 to 8,
wherein the venetoclax is administered with a daily dose of from 20 mg to 1000 mg, preferably from 50 mg to 600 mg, more preferably from 300 mg to 600 mg, even more preferably from 400 mg to 600 mg, even more preferably 400 mg or 600 mg.

10. The HDM2-p53 interaction inhibitor drug for use according to any one of claims 1 and 4 to 9, the BCL2 inhibitor for use according to any one of claims 2 and 4 to 9, or the hypomethylating agent for use according to any one of claims 3 to 9,
wherein the HDM201 is administered on each of the first 5 days of a 28 days treatment cycle followed by a drug-administration-free period from day 6 until day 28.

11. The HDM2-p53 interaction inhibitor drug for use according to any one of claims 1 and 4 to 10, the BCL2 inhibitor for use according to any one of claims 2 and 4 to 10, or the hypomethylating agent for use according to any one of claims 3 to 10, wherein the HDM201 is present as a succinic acid co-crystal, preferably as a 1:1 (molar ratio) succinic acid : HDM201 co-crystal.
